# EUROPEAN PATENT APPLICATION

(11) **EP 1 090 592 A1**
(43) Date of publication of application: **11.04.2001**
(21) Application number: 00308735.0
(22) Date of filing: 04.10.2000
(51) Int. Cl.: A61B 17/072

(54) **Surgical stapler having two staple forming surfaces**

(30) Priority: 05.10.1999 US 412724
(71) Applicant: ETHICON ENDO-SURGERY, Cincinnati, Ohio 45242 (US)
(72) Inventor: Nalagatla, Anil K., Mason, Ohio 45040 (US); Neurohr, Mark A., Cincinnati, Ohio 45204 (US); Setser, Michael E., Burlington, Kentucky 41005 (US)
(74) Representative: Mercer, Christopher Paul

(57) **Abstract**

A surgical stapling instrument for clamping and stapling tissue is disclosed having a staple cartridge having a first tissue contact surface thereon, a plurality of staple slots in the first tissue contact surface, and staples contained within the plurality of staple slots. An anvil having a longitudinal axis and first and second staple forming surfaces thereon is coupled to the staple cartridge. The first and second staple forming surfaces are spaced from each other and have a plurality of staple pockets embedded therein. The first and second staple forming surfaces of the anvil face the first tissue contact surface of said staple cartridge, and the staple pockets embedded in the first and second staple forming surfaces are aligned with the staple slots within the first tissue contact surface. The staple cartridge and the anvil are moveable relative to each other from an open position for positioning tissue therebetween to a closed position generally parallel to each other for clamping the tissue. When the staple cartridge and anvil are moved to the closed position, the first staple forming surface of the anvil is spaced a first distance away from the first tissue contact surface of the staple cartridge and the second staple forming surface of the anvil is spaced a second distance away from the first tissue forming surface of the staple cartridge. The first distance is different from the second distance, and the first and second distances are taken perpendicularly to the anvil longitudinal axis.

## Description

### Field of the Invention

The present invention relates, in general, to a surgical stapling instrument and, more particularly, to a surgical stapling instrument having certain spatial relationships between the contact surfaces of the cartridge and anvil of the tissue fastening assembly of the instrument.

### Background of the Invention

Surgical stapling instruments are well known in the art and are frequently used to suture or resect a portion of body tissue such as lung, intestine, stomach, uterus, or esophagus. Stapling tissue was found to be quick and effective, and dramatically reduced the difficulty and length of many surgical procedures. Surgical stapling instruments provided advantages over conventional suturing by rapidly stapling a large portion of body tissue, producing a hemostatic and pneumostatic barrier, and clamping and stapling the walls of a hollow organ or vessel together. These advantages moved surgical stapling instruments to the forefront of useful surgical tools.

Surgical stapling instruments are of two general categories; those suited for open surgical procedures and those suited for endoscopic procedures. Of special interest are endoscopic surgical stapling instruments. The endoscopic surgical stapling instrument is used in minimally invasive surgical procedures wherein the surgery is performed through a small number of small diameter surgical access ports rather than through a large opening or incision within the patient. In a typical endoscopic surgery, the abdominal cavity is insufflated with an inert gas and surgical access ports are inserted into the patient. Endoscopic stapling instruments, such as an endoscopic linear cutter, are inserted into the access ports and the surgical procedure is performed through these access ports. Endoscopic surgery is sometimes referred to as "keyhole surgery" wherein the access ports are the "keyholes" through which the surgery is performed. As a consequence of the access port ("keyhole") size, endoscopic linear cutters are characterized by a handle, a small diameter elongated shaft that forms a gas tight seal with the access port, and an elongated tissue fastening assembly of a diameter to fit within the access port.

The stapling tissue fastening assembly of the endoscopic linear cutter generally has an elongated staple cartridge filled with four or six longitudinal rows of staples and an anvil for forming the staples. Tissue is placed between the open anvil and the staple cartridge and a bite of tissue is tightly compressed or clamped when the anvil is closed. Staples are ejected from the staple cartridge into the clamped tissue and are formed in the clamped tissue by contact with a staple forming surface on the anvil. After staples are formed, the stapled tissue is cut and the anvil is opened to release the stapled tissue. This type of stapling and cutting action is well suited for the resection or removal of a portion of diseased tissue. Linear cutters are well, known in the art and are described in many U.S. Patents such as U.S. Patent No. 5,040,715 by Green et al., U.S. Patent 5,307,976 by Olsen et al., and in U.S. Patent No. 5,465,895 by Knodel et al.

Whereas these linear cutters were of great value for many types of surgery, surgeons demanded an increase in the tissue bite length from 35mm to 45mm. The increase in bite length was welcomed by thoracic surgeons and was useful for stapling stiffer tissues such as the esophagus. However, the incompressibility of the stiffer tissues and the increased length of the tissue bite resulted in a greater cantilever load on the anvil. To counteract the increased tissue loading and the resulting increased anvil deflection, additional anvil stiffening was needed. The initial solution was to simply increase the height or thickness of the anvil to increase the anvil stiffness at a cost of increasing the size or diameter of the end effector. Unfortunately, the increased size of the tissue fastening assembly required an increase in the size of the access port or trocar cannula and this was unacceptable to some surgeons.

What was needed was an endoscopic stapling instrument having an increased tissue bite length and a stiffer anvil with no increase in the diameter of the end effector. In order to remain cost effective, a change of materials was not acceptable.

The additional stiffening would be optimally accomplished by the addition of additional material to the anvil in the area between the cartridge and the anvil. The addition of material or features to this portion of an anvil is well known in the art. The addition of a pair of tissue gripping ridges having serrated surfaces or teeth for gripping tissue is taught by Williamson, IV et al. in U.S. Patent No. 5,452,837. The serrated teeth are located on both the anvil and staple cartridge and provide little stiffening due to the gaps between the serrated teeth.

Tim Knodel et al. in U.S. Patent No. 5,697,542, U.S. Patent No. 5,769,303, U.S. Patent No. 5,779,131, and U.S. Patent No. 5,779,132 teach bringing the anvil into intimate contact with the staple cartridge prior to insertion of the tissue fastening assembly into a trocar. Closing the tissue fastening assembly of the surgical stapling instrument prior to insertion reduces the overall closed height of the end effector. However, Tim Knodel et al. does not teach stiffening the anvil in the areas of tissue contact.

An anvil of "T" shaped cross section is disclosed by Yates et al. in U. S. Patent 5,709,680. The anvil is used in an electrosurgical instrument and is formed from a ceramic insulative material having a ridge or rib that extends inwardly towards the staple cartridge to form the vertical of the "T" cross section. Yates et al. teaches the use of the rib as a tissue compression member but does not teach the use of the rib as a stiffening member. Unfortunately, the central rib has no staple forming surface and reduces the number of longitudinal rows of staples that can be applied to tissue from the conventional four or six rows down to two rows.

At present, there is no known surgical instrument that can meet all of the needs outlined above. These and other advantages will become more apparent from the following detailed description and drawings.

### Summary of the Invention

The present invention is a surgical stapling instrument having a tissue fastening assembly for clamping and stapling tissue. The tissue fastening assembly includes a staple cartridge having a first tissue contact surface thereon, a plurality of staple slots within the first tissue contact surface, and containing staples within the plurality of staple slots.

An anvil is coupled to the staple cartridge. The anvil has a longitudinal axis, a first, and a second staple forming surfaces thereon. The first and a second staple forming surfaces are spaced from each other and have a plurality of staple pockets embedded in the staple forming surfaces. The staple forming surfaces of the anvil face the first tissue contact surface of the staple cartridge. The staple pockets in the staple forming surfaces are aligned with the staple slots in the first tissue contact surface for forming a plurality of rows of staples therebetween.

The staple cartridge and the anvil are moveable relative to each other from an open position for positioning tissue therebetween, to a closed position generally parallel to each other for clamping the tissue. When the staple cartridge and the anvil are moved to the closed position, the first staple forming surface of the anvil is spaced a first distance away from the first tissue contact surface of the staple cartridge, and the second staple forming surface of the anvil is spaced a second distance away from the first tissue forming surface of the staple cartridge. The first distance being different from the second distance and the first and second distances being taken perpendicularly to the anvil longitudinal axis.

Significantly, the novel surgical stapling instrument for clamping and stapling tissue during a surgical procedure enhances the surgeon's capabilities during an operative procedure. Specifically, the difference in distances between the first tissue contact surface of the cartridge and the first and second staple forming surfaces of the anvil stiffens the anvil and consequently enables the medical designer of the instrument to increase the length of the anvil for a longer tissue bite (and concomittently decrease operative time). The anvil is stiffened without the need for increasing the diameter of the tissue fastening assembly. This is especially significant, as the trend is towards the same or smaller diameter surgical instruments and access ports to reduce trauma and wound size at the access port insertion point. Consequently, the surgeon is provided with an improved surgical stapling instrument with an increased staple line length, which reduces operative time. This benefit can be achieved without an associated increase in the diameter of the instrument or access port.

In an alternate preferred embodiment of the present invention, the tissue fastening assembly of the surgical stapling instrument includes a staple cartridge having first and second tissue contact surfaces thereon. The first tissue contact surface has a first plurality of staple slots in the first tissue contact surface and contains staples within. The second tissue contact surface is spaced from the first tissue contact surface and is recessed within the staple cartridge so as to form a cartridge slot therein.

An anvil of the alternate preferred embodiment is coupled to the staple cartridge. The anvil has a longitudinal axis, a first, and a second staple forming surfaces thereon. The first and a second staple forming surfaces are spaced from each other and have a plurality of staple pockets embedded in the staple forming surfaces. The first and second staple forming surfaces of the anvil face the first and second tissue contact surfaces of the staple cartridge respectively. The staple pockets in the first staple forming surface are aligned with the staple slots in the first tissue contact surface for forming a plurality of rows of staples therebetween. The second staple forming surface is aligned with the cartridge slot.

The staple cartridge and the anvil of the alternate preferred embodiment are moveable relative to each other from an open position for positioning tissue therebetween, to a closed position generally parallel to each other for clamping the tissue. When the staple cartridge and the anvil are moved to the closed position, the first staple forming surface of the anvil is spaced a third distance away from the first tissue contact surface of the staple cartridge. The second staple forming surface of the anvil is spaced a fourth distance away from the second tissue contact surface of the staple cartridge. The third distance being substantially the same as the fourth distance and the third and fourth distances being taken perpendicularly to the anvil longitudinal axis.

In yet another embodiment of the preferred invention, the second staple forming surface is located on a rib of the anvil and the cartridge has a cartridge slot. Both the anvil and the cartridge slot are substantially parallel to the anvil longitudinal axis. Consequently, when the anvil is closed on the staple cartridge, the rib of the staple cartridge is received within the cartridge slot. The reception of the rib within the cartridge slot aligns the staple pockets of the anvil to the staples in the cartridge in a direction lateral to the anvil longitudinal axis and perpendicular to the first staple forming surface of the anvil. Additionally, the reception of the rib in the cartridge slot restrains the anvil from motion in a direction lateral to the anvil longitudinal axis and perpendicular to the first staple forming surface of the anvil.

For a better understanding of the invention and its unique features, reference is made to the accompanying drawings and descriptive matter in which the preferred invention is fully described.

### Brief Description of the Drawings

The novel features of the invention are set forth with particularity in the appended claims. The invention itself, however, both as to organization and methods of operation, together with further objects and advantages thereof, may best be understood by reference to the following description, taken in conjunction with the accompanying drawings in which:
FIG. 1 is an isometric view of a surgical stapling instrument having a distal tissue fastening assembly;
FIG. 2 is an isometric view of a distal end of the surgical stapling instrument of FIG. 1 wherein the tissue fastening assembly has a six-row staple cartridge and an anvil, and the anvil is shown sectioned and rotated for clarity;
FIG. 3 is an isometric view of the distal end of the surgical stapling instrument of FIG. 1 wherein the distal end has a four-row staple cartridge and an anvil, and the anvil of the tissue fastening assembly is sectioned and rotated for clarity.
FIG. 4 is an isometric view of a tissue portion placed between the open anvil and stapling cartridge of the surgical stapling instrument of FIG. 1;
FIG. 5 is an isometric view of a tissue portion clamped between the cartridge and the closed anvil of the surgical stapling instrument of FIG. 4;
FIG. 6 is an end view, in cross section, of the cartridge and the closed anvil of FIG 5 wherein the cartridge is the six-row cartridge of FIG. 2;
FIG. 7 is an end view, in cross section, of the closed tissue fastening assembly of FIG 5 wherein the cartridge is the four-row cartridge of FIG. 3.

### Detailed Description of the Invention

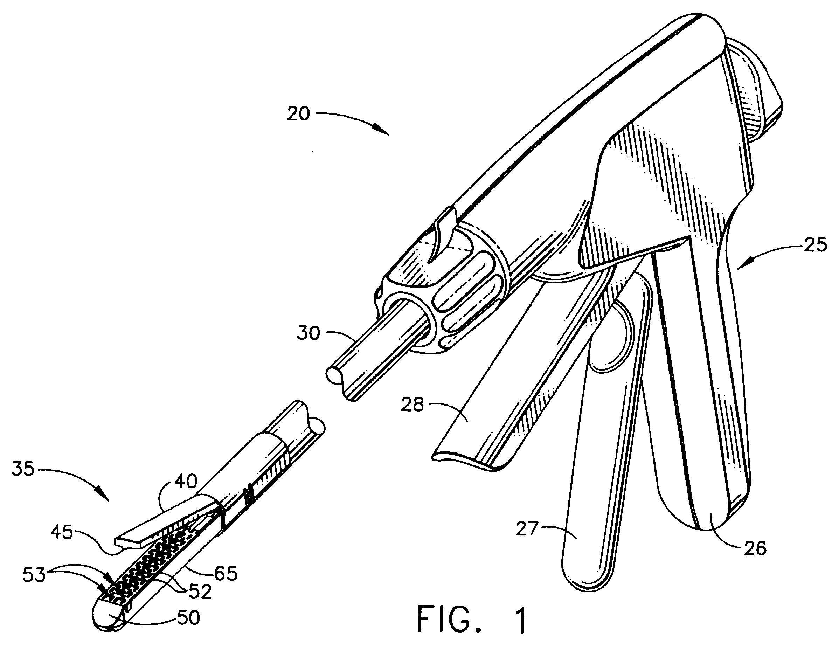

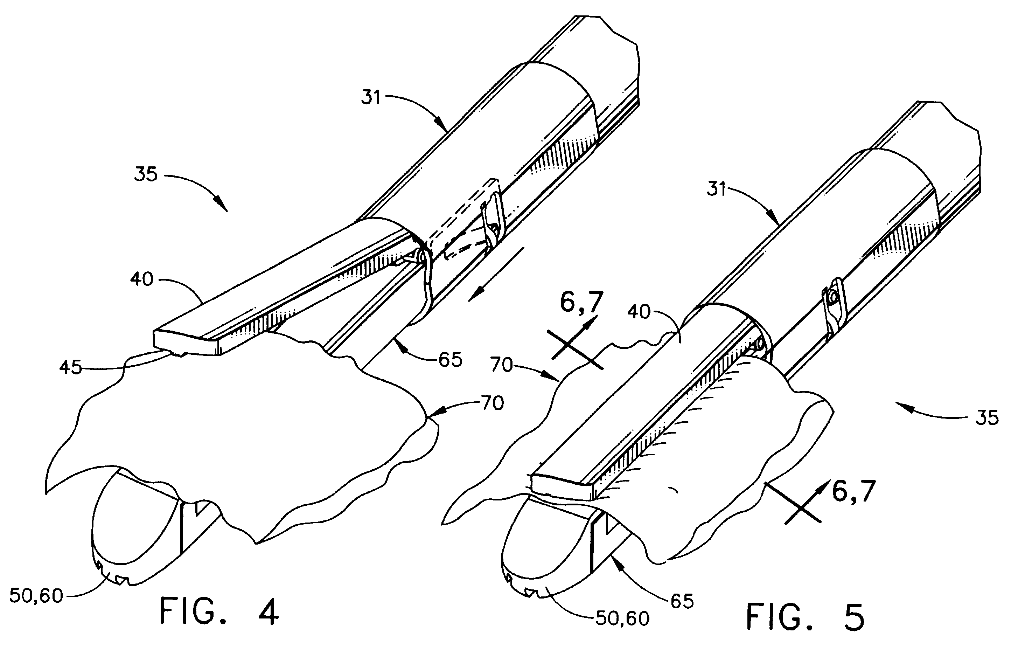

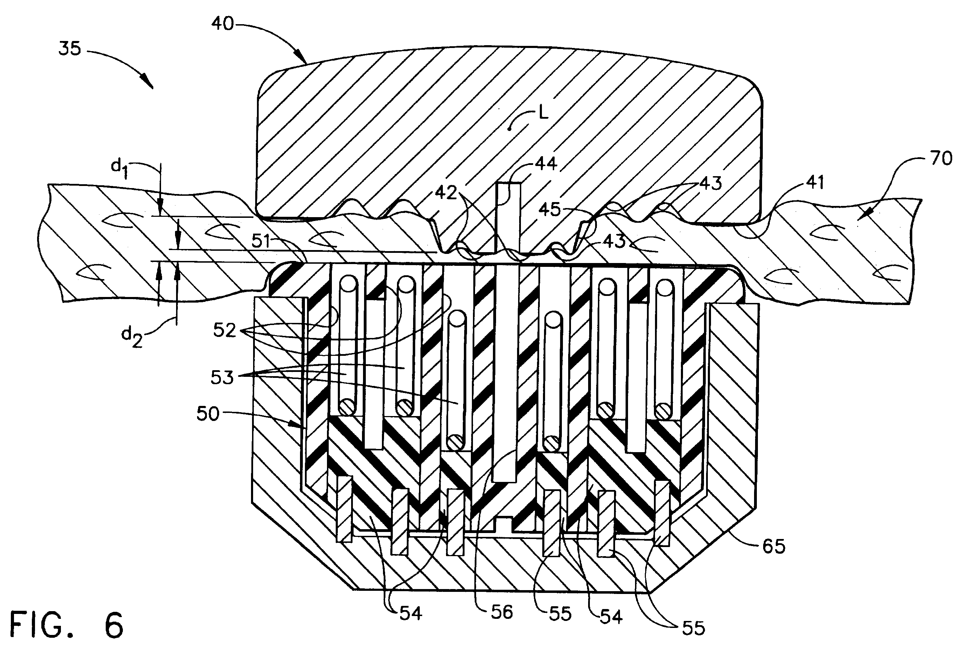

FIGS. 1-7 show a surgical stapling instrument 20 of the present invention for clamping and stapling body tissue. As sown in FIG. 1, the surgical stapling instrument 20 has a handle 25 for the operator to grasp, an elongated shaft 30 extending distally from the handle 25, and an tissue fastening assembly 35 extending distally from the shaft 30. The tissue fastening assembly 35 has a moveable anvil 40 and a fixed channel 65 that holds a removable six-row staple cartridge 50 (FIGS. 1 and 2, and FIGS. 4-6). The six-row staple cartridge 50 has a first tissue contact surface 51 and six longitudinal rows of staple slots 52 containing staples 53 therein (see FIG. 6). A rib 45 that extends inwardly towards the six-row staple cartridge 50 stiffens the anvil 40 of the present invention. Anvil 40 is operably attached to a closure trigger 27 and movement of the closure trigger 27 towards a grip 26 of the handle 25 moves the anvil 40 from the open position of FIG. 1 to the closed position of FIG. 5 to clamp tissue 70 therebetween. Movement of the firing trigger 28 ejects the staples 53 from the cartridge 50 into the clamped tissue 70, forms the staples 53 against the anvil 40, and moves a knife between the inner most rows of staples 53 (not shown).

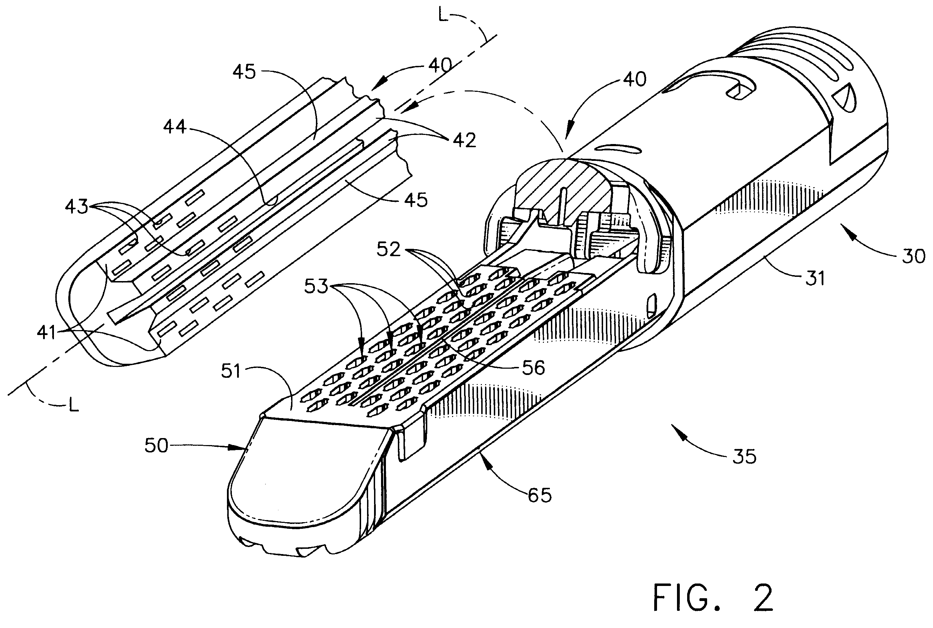

FIG. 2 is an isometric view of the tissue fastening assembly 35 of FIG. 1 with the six-row cartridge 50 installed and with the anvil 40 broken and rotated for clarity. As shown, anvil 40 has a "T" shaped cross section and a longitudinal axis "L" that is generally parallel to the shaft 30 when the anvil 40 is closed (see FIG. 5). Rib 45 is parallel to the longitudinal axis "L" and extends inwardly from a first staple forming surface 41 that flanks both sides of longitudinal rib 45. A second staple forming surface 42 is located on rib 45 and is spaced away from the first staple forming surface 41 a distance that is between about 0.010 and about 0.060 inches and is preferably about 0.025 inches. A knife slot 44 bisects the rib 45 and the second staple forming surface 42 and is provided for the passage of a tissue-cutting knife (not shown). The first and second staple forming surfaces 41 and 42 have six rows of staple pockets 43 formed therein such that closure of the anvil 40 onto the six-row cartridge 50 aligns the staple pockets 43 of the anvil 40 with the staples 53 and staple slots 52 of the six-row cartridge 50 (FIG. 6). A cartridge knife slot 56is provided for passage of the knife through the cartridge 50.

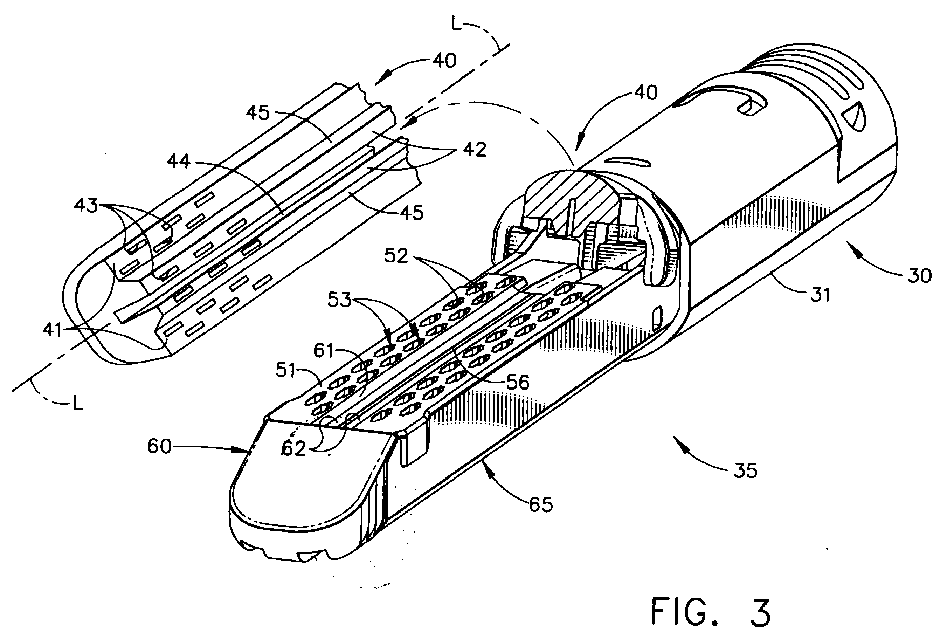

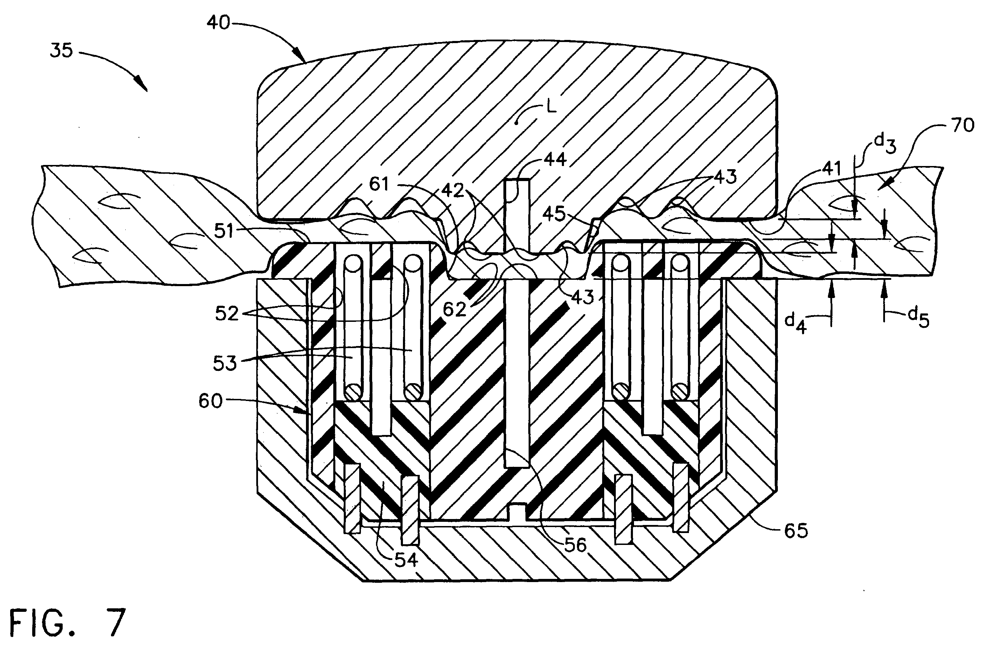

FIG. 3 is identical to that of FIG. 2 except the removable six-row cartridge 50 has been replaced with a removable four-row cartridge 60. Four-row cartridge 60 is generally similar to the previously described six-row staple cartridge 50 but has a recessed cartridge slot 61 replacing the two innermost rows of staple slots 52 and staples 53. The cartridge slot 61 has a second tissue contact surface 62 that is both recessed into the four-row cartridge 60 and spaced away from the first tissue contact surface 51. When the anvil 40 is closed, the cartridge slot 61 receives the rib 45 of the anvil 40 and longitudinally aligns the anvil 40 to the four-row cartridge 60 (FIG. 7).

FIG. 4 shows the placement of a portion of tissue 70 between the open anvil 40 and the cartridge 50 or 60 of the surgical stapling device of FIG. 1. FIG. 5 shows the anvil 40 in the closed position clamping the portion of tissue 70 within the tissue fastening assembly 35. FIGS. 6 and 7 are cross-sections of the tissue fastening assembly 35 of FIG. 5 with FIG. 6 showing tissue 70 clamped between the anvil 40 and a six-row cartridge 50, and FIG. 7 showing tissue 70 clamped between the anvil 40 and a four-row cartridge 60.

The cross section of FIG. 6 clearly shows the "T" shaped cross section of the anvil 40 and the relationship of the anvil 40 features to the features and internal components of the six-row cartridge 50 when the anvil is closed. Closure of the anvil 40 aligns the staples 53 with the staple pockets 43. The six-row staple cartridge 50 is of conventional endocutter design having six laterally spaced longitudinal rows of staple slots 52 and staples 53 therein for stapling tissue 70. Staple drivers 54 are slidably located within the staple slots 52 and are initially positioned just below the staples 53. Wedges 55 are longitudinally moveable within the cartridge and distal movement of the wedges 55 engages the staple drivers 54 to effect the firing of the staples 53 towards the anvil 40. It is important to note that when the anvil 40 is closed, the first staple forming surface 41 of the anvil 40 is spaced a first distance d₁ from the first tissue contact surface 51 of the six-row cartridge 50, and that the second staple forming surface 42 is spaced a second distance d₂ from the first tissue contact surface 51 of the six-row cartridge 50, and that d₁ is different from d₂. The two innermost rows of staples 53 and staple drivers 54 are recessed farther into the six-row staple cartridge 50 than the outermost four rows to space each of the staples 53 and staple drivers 54 a generally equal distance from their respective staple pockets 43 in the first and second staple forming surfaces 41 and 42. It is also of note that the unfired staples 53 and staple drivers 54 are generally equidistant from the first and the second staple forming surfaces 41 and 42 of the closed anvil 40.

The cross section of FIG. 7 shows tissue 70 clamped between the anvil 40 and the four-row staple cartridge 60. As described previously, the four-row staple cartridge 60 is generally similar to the six-row staple cartridge 50 but has the two inner rows of staples 53 and staple drivers 54 and wedges 55 replaced by a cartridge slot 61 that is parallel to the longitudinal axis of the anvil 40. Cartridge slot 61 has the second tissue contact surface 62 that is recessed within said staple cartridge a distance d₅ between about 0.010 and about 0.060 inches and preferably about 0.025 inches. As shown, rib 45 of the closed anvil is received within the cartridge slot 61 of the four-row cartridge 60 and laterally aligns the staples 53 to the staple slots 43 of the anvil 40. Additionally, the reception of the rib 45 within the cartridge slot 61 constrains the anvil 40 from lateral motion. It is important to note that with the four-row cartridge, when the anvil 40 is closed, the first staple forming surface 41 of the anvil 40 is spaced a first distance d₃ from the first tissue contact surface 51 of the four-row cartridge 60, and that the second staple forming surface 42 is spaced a second distance d₄ from the second tissue contact surface 62 of the four-row cartridge 60, and that d₃ and d₄ are substantially the same.

While preferred embodiments of the present invention have been shown and described herein, it will be obvious to those skilled in the art that such embodiments are provided by way of example only. Numerous variations, changes, and substitutions will now occur to those skilled in the art without departing from the invention. Accordingly, it is intended that the invention be limited only by the spirit and scope of the appended claims.

## Claims

1. A surgical stapling instrument comprising a tissue fastening assembly for clamping and stapling tissue, said tissue fastening assembly including:
a staple cartridge having a first tissue contact surface thereon, said first tissue contact surface having a plurality of staple slots in said first tissue contact surface and containing staples therein;
an anvil coupled to said staple cartridge, said anvil having a longitudinal axis and first and second staple forming surfaces thereon spaced from each other, said staple forming surfaces having a plurality of staple pockets embedded in said staple forming surfaces, said staple forming surfaces facing said first tissue contact surface of said staple cartridge, said staple pockets in said staple forming surfaces being aligned with said staple slots in said first tissue contact surface for forming a plurality of rows of staples therebetween;
wherein said staple cartridge and said anvil are moveable relative to each other from an open position for positioning tissue therebetween to a closed position generally parallel to each other for clamping the tissue, and when said staple cartridge and anvil are moved to the closed position, said first staple forming surface of said anvil is spaced a first distance away from said first tissue contact surface of said staple cartridge, and said second staple forming surface of said anvil is spaced a second distance away from said first tissue forming surface of said staple cartridge, the first distance being different from the second distance, and the first and second distances being taken perpendicularly to the anvil longitudinal axis.

2. The instrument of claim 1 wherein the difference between the first and second distances is between 0.010 and 0.060 inches and is preferably about 0.025 inches.

3. The instrument of claim 1 or claim 2 wherein when the anvil is closed, each of said staples in said staple cartridge is aligned with its respective staple pocket in said anvil, and the distance between each of said staples and its respective staple pocket, in a direction taken perpendicularly to the anvil longitudinal axis, is substantially the same.

4. A surgical stapling instrument comprising a tissue fastening assembly for clamping and stapling tissue, said tissue fastening assembly including:
a staple cartridge having first and second tissue contact surfaces thereon, said first tissue contact surface having a first plurality of staple slots in said first tissue contact surface and containing staples therein, said second tissue contact surface spaced from said first tissue contact surface and recessed within said staple cartridge so as to form a cartridge slot therein;
an anvil coupled to said staple cartridge, said anvil having a longitudinal axis and first and second staple forming surfaces thereon spaced from each other, said staple forming surfaces having a plurality of staple pockets embedded in said staple forming surfaces, said first and second staple forming surfaces facing said first and second tissue contact surfaces of said staple cartridge respectively, said staple pockets embedded in said first staple forming surface being aligned with said staple slots in said first tissue contact surface for forming a plurality of rows of staples therebetween, and said second staple forming surface being aligned with said cartridge slot;
wherein said staple cartridge and said anvil are moveable relative to each other from an open position for positioning tissue therebetween to a closed position generally parallel to each other for clamping the tissue, and when said staple cartridge and anvil are moved to the closed position, said first staple forming surface of said anvil is spaced a third distance away from said first tissue contact surface of said staple cartridge and said second staple forming surface of said anvil is spaced a fourth distance away from said second tissue contact surface of said staple cartridge, the third distance being substantially the same as the fourth distance, and the third and fourth distances being taken perpendicularly to the anvil longitudinal axis.

5. The instrument of claim 4 wherein said second tissue contact surface of said cartridge is spaced away from said first tissue contact surface of said cartridge a distance between 0.010 and 0.060 inches, preferably about 0.025 inches.

6. The instrument of any one of claims 1 to 5 wherein said second staple forming surface is a rib.

7. The instrument of claim 6 wherein said rib is substantially parallel to the anvil longitudinal axis.

8. The instrument of claim 6 or claim 7 wherein said cartridge slot is substantially parallel to the anvil longitudinal axis.

9. The instrument of claim 7 when dependent on claim 4 wherein, when said anvil is closed on said cartridge, said rib of said cartridge is received within said cartridge slot of said cartridge to align said staple pockets of said anvil to said staples in said cartridge in a direction lateral to said anvil longitudinal axis and perpendicular to said first staple forming surface of said anvil.

10. The instrument of any one of claims 6 to 9 wherein said anvil contains a knife slot substantially parallel to said anvil longitudinal axis, said knife slot bisecting said rib and said second staple forming surface.
